Europäisches Patentamt

European Patent Office

Office européen des brevets

(19)

(11) Numéro de publication : **0 599 711 A1**

(12) **DEMANDE DE BREVET EUROPEEN**

(21) Numéro de dépôt : **93402818.4**

(22) Date de dépôt : **19.11.93**

(51) Int. Cl.⁵ : **C02F 3/32, C12M 1/00**

(30) Priorité : **23.11.92 FR 9214037**

(43) Date de publication de la demande :
**01.06.94 Bulletin 94/22**

(84) Etats contractants désignés :
**BE CH DE ES GB IT LI**

(71) Demandeur : **COMMISSARIAT A L'ENERGIE ATOMIQUE**
**31-33, rue de la Fédération**
**F-75015 Paris (FR)**

(72) Inventeur : **Chaumont, Daniel**
**Rue de la Bergerie,**
**Allée des Ecureuils**
**F-13770 Venelles (FR)**
Inventeur : **Thepenier, Catherine**
**Les Restanques de la Tomassine**
**F-04100 Manosque (FR)**
Inventeur : **Gudin, Claude**
**4, rue Sainte Anne,**
**Le Parc**
**F-13100 Aix en Provence (FR)**

(74) Mandataire : **Dubois-Chabert, Guy et al**
**c/o BREVATOME**
**25, rue de Ponthieu**
**F-75008 Paris (FR)**

(54) **Dispositif d'épuration d'un effluent liquide chargé en polluants et procédé d'épuration de cet effluent.**

(57) L'invention concerne un dispositif d'épuration d'un effluent liquide chargé en polluants, notamment en métaux et/ou en radionucléides, ainsi qu'un procédé d'épuration de cet effluent.

Le but de l'invention est de réaliser un dispositif d'épuration fonctionnant en continu.

Ce but est atteint à l'aide d'un dispositif comprenant une enceinte de confinement (1) contenant un support (7) dans lequel sont immobilisées des cellules vivantes de micro-organismes photosynthétiques, ce support étant réalisé dans un matériau transparent aux rayonnements lumineux, ce dispositif comprenant également des moyens d'introduction et de mise en circulation à l'intérieur de l'enceinte (1), de l'effluent à traiter et d'un milieu de culture.

FIG. 1

EP 0 599 711 A1

Jouve, 18, rue Saint-Denis, 75001 PARIS

La présente invention concerne un dispositif d'épuration d'un effluent liquide chargé en polluants, notamment en métaux et/ou en radionucléides, ainsi qu'un procédé d'épuration de cet effluent.

De nombreux procédés industriels entraînent la formation d'effluents contenant des métaux tels que le cuivre ou le nickel par exemple, ou des métaux lourds tels que le cadmium ou le mercure. D'autres procédés entraînent également la formation de radionucléides, tels que l'uranium. Toutes ces substances constituent une source importante de pollution et il est primordial de les traiter avant de les rejeter dans le milieu naturel.

Les métaux contenus dans un effluent liquide se présentent soit comme ions du métal libre, soit combinés sous de nombreuses formes chimiques. La forme ionique du métal libre est en général la forme la plus toxique alors que la forme combinée l'est moins. On peut même considérer que la toxicité d'un élément métallique décroît avec l'importance du complexage matière organique/métal. Le complexage du métal s'effectue généralement avec des amino-acides, des polypeptides, des acides gras ou des polysaccharides. Ces composés présentent en effet la caractéristique d'être chargés négativement et de constituer ainsi un site d'ancrage pour les ions métalliques qui sont chargés positivement.

On connaît déjà d'après l'art antérieur plusieurs procédés permettant d'épurer un effluent contenant des métaux ou des radionucléides, en utilisant des bactéries ou des micro-algues.

L'un de ces procédés consiste à utiliser des biofiltres qui sont des réacteurs obscurs, remplis d'anneaux sur lesquels se fixent les bactéries. Ces anneaux ont pour but d'augmenter la surface et le temps de contact entre les bactéries qui y sont fixées et l'effluent qui traverse le biofiltre. Ces bactéries peuvent piéger certains éléments polluants, ou se nourrir et se développer à partir de ceux-ci.

Toutefois, si ce type de procédé est mal contrôlé, il peut aboutir à remplacer une contamination chimique par une contamination bactérienne qui peut parfois être tout aussi dangereuse. En outre, l'affinité des bactéries pour ces métaux peut entraîner une destruction de leurs parois, ce qui leur fait perdre leurs propriétés de fixation et entraîne leur mort assez rapidement.

Par ailleurs, il a été démontré que les micro-algues sont de très puissants absorbeurs d'ions métalliques, notamment grâce à leur grande surface cellulaire chargée négativement. En outre, certaines espèces, telles que par exemple _Porphyridium cruentum_, produisent et excrètent de grande quantité de polysaccharides pouvant représenter jusqu'à 50% de la quantité totale des matières organiques produites par ces micro-algues. Ces polysaccharides sont polyanioniques et présentent des sites $COO^-$ et $SO_3^-$. C'est justement cette caractéristique qui permet de piéger les métaux lourds et les radionucléides. Il s'agit d'un phénomène rapide et réversible se réalisant en quelques minutes.

On connaît déjà d'après l'art antérieur un procédé d'épuration d'eaux usées utilisant des micro-algues, par la technique dite de lagunage. Cette technique de lagunage consiste à cultiver dans des bassins à ciel ouvert, un mélange de plusieurs espèces de micro-algues. Ces dernières se développent grâce aux éléments nutritifs et en particulier à l'azote fourni par les effluents à traiter. Par une réaction de photosynthèse, ces micro-algues produisent de l'oxygène qui est ensuite utilisé par les bactéries se trouvant également dans ce bassin de lagunage.

Cette technique d'épuration est mixte, puisqu'elle fait appel simultanément aux métabolismes respectifs des algues et des bactéries, ce qui permet d'épurer des polluants de natures diverses.

Toutefois, cette technique à ciel ouvert présente l'inconvénient de ne pas permettre le contrôle du développement sélectif d'une espèce déterminée de micro-algue. En conséquence, il n'est pas possible de favoriser l'extraction spécifique d'un élément métallique ou d'un radionucléide.

On connaît également d'après l'art antérieur un procédé exploité industriellement par les Sociétés américaines BIORECOVERY SYSTEM INC. et GEOMICROBIAL TECHNOLOGIES INC., pour accumuler de l'or, du mercure ou de l'uranium. Dans cette technique, des cellules mortes de micro-algues de la famille des chlorophycées (_Chlorella vulgaris_ et _Chlorella regularis_) sont piégées dans un substrat opaque, se présentant sous la forme d'une colonne de gel de silice. L'ensemble constitue un biofiltre. L'effluent à traiter traverse la colonne de gel de silice et les ions métalliques se fixent sur les parois cellulaires des micro-algues par des forces électrostatiques. Ils occupent les sites négatifs de ces parois, constitués par exemple par des ions carboxylates qui sont des agents chelatants. Ainsi, ces micro-algues peuvent "bio-accumuler" jusqu'à 15% de leur matière sèche en uranium et jusqu'à 10% de leur matière sèche en or. La désorption des polluants s'effectue en modifiant le pH du milieu dans lesquelles se trouvent les micro-algues. Celles-ci, sous l'action du stress provoqué par la modification de pH, relâchent dans la circulation les métaux lourds et les radionucléides qu'il est alors possible de faire éluer.

D'après les inventeurs de cette technique, ce procédé permet de piéger sélectivement certains métaux lourds et non les ions comme le calcium par exemple, qui entrent souvent en compétition avec ces métaux. Cette technique est donc plus performante que celle utilisant les résines échangeuses d'ions.

Toutefois, ce procédé présente des inconvénients. Plus précisément, le biofiltre ne peut être utilisé. qu'un nombre limité de fois. En effet, les cellules de micro-algues étant mortes, leurs parois ne peuvent piéger qu'une certaine quantité de polluants et lorsque tous les agents chelatants ont piégé un ion métallique, le biofiltre doit être obligatoirement rechargé en nouvelles cellules de micro-algues. Ceci entraîne des manipulations longues et complexes et une période d'inactivation du biofiltre.

On connaît enfin par un article paru dans le Compte rendu de l'Académie des Sciences de Paris, le 6 juillet 1981, série III, 35-37, intitulé "Production de polysaccharides sulfatés par un biophotoréacteur à cellules immobilisées de <u>Porphyridium cruentum</u>", rédigé par de C. GUDIN et D. THOMAS, un photobioréacteur permettant de cultiver en continu des cellules vivantes de <u>Porphyridium cruentum</u>. Cette culture s'effectue sous lumière artificielle, en présence d'un apport constant en $CO_2$ et en l'absence d'azote. Ces cellules excrètent en continu un polysaccharide capsulaire sulfaté soluble. Toutefois, ce document ne prévoit aucune possibilité de traitement d'un effluent pollué.

La présente invention a justement pour but de remédier aux inconvénients des dispositifs d'épuration d'effluents de l'art antérieur.

A cet effet, l'invention concerne un dispositif d'épuration d'un effluent liquide chargé en polluants, notamment en métaux et/ou en radionucléïdes, comprenant une enceinte de confinement (1), destinée à recevoir des cellules de micro-organismes photosynthétiques, aptes à piéger lesdits polluants, des moyens (31) d'introduction de l'effluent à traiter, des moyens (35) de mise en circulation de cet effluent à travers ladite enceinte (1), des moyens de prélèvement (33, 65) de l'effluent traité, liquide et des moyens de prélèvement (33) des polluants, et des moyens (23) d'introduction de $CO_2$ à l'intérieur de l'enceinte.

Selon les caractéristiques de l'invention, les cellules de micro-organismes sont des cellules vivantes, immobilisées sur un support (7), ce support (7) occupant au moins partiellement l'intérieur de l'enceinte (1) et étant traversé par l'effluent à traiter et le dispositif comprend des moyens d'introduction (21) et des moyens de mise en circulation (23) d'un milieu de culture nutritif liquide à travers ledit support (7), les parois de l'enceinte (1) ainsi que le support (7) des cellules étant réalisés dans un matériau transparent aux rayonnements lumineux.

Ainsi, contrairement au dispositif d'épuration de l'art antérieur, (dispositifs de BIORECOVERY SYSTEM INC. et GEOMICROBIAL TECHNOLOGIES INC.), les micro-organismes utilisés ici sont des cellules vivantes qui produisent continuellement les métabolites ou agents chélatants capables de fixer les ions polluants. En conséquence, ce dispositif est utilisable en permanence et permet d'effectuer un traitement d'épuration en continu, sans avoir à recharger périodiquement le support en cellules de micro-organismes. Le fonctionnement du dispositif s'en trouve donc simplifié. Ce support est choisi de façon à être traversé facilement par l'effluent à traiter et par le milieu de culture.

De façon avantageuse, les moyens de mise en circulation assurent le déplacement de l'effluent et du milieu de culture du bas en haut du support d'immobilisation des cellules, ledit support ne s'étendant que sur une partie de la hauteur totale de l'enceinte de confinement de façon à ménager un espace libre inférieur et un espace libre supérieur à l'intérieur de ladite enceinte. En outre, un puits formant trop-plein est prévu dans le support pour relier entre eux lesdits deux espaces libres, le milieu de culture et l'effluent se déversant dans ce puits après être parvenus dans l'espace libre supérieur.

Ce dispositif permet d'assurer une boucle de circulation du milieu de culture et de l'effluent à traiter à travers le support. L'effluent passe ainsi autant de fois que nécessaire à travers le support jusqu'à ce que tous les métaux et radionucléïdes soient accumulés par les cellules des micro-organismes.

De façon avantageuse, au moins un tube est placé à l'intérieur de l'enceinte de confinement et traverse celle-ci de part en part, ce tube permettant le passage des rayons lumineux provenant de l'extérieur. De préférence, une lampe est placée à l'intérieur dudit tube.

Ainsi, la culture de micro-organismes est éclairée au maximum, ce qui permet de réaliser complétement la réaction de photosynthèse et d'assurer de bonnes conditions de culture et de reproduction des micro-organismes.

Selon une caractéristique avantageuse de l'invention, le dispositif comprend, montés en série, un capteur de mesure du pH régnant à l'intérieur de l'enceinte de confinement, un analyseur de pH, un régulateur de pH et une vanne agissant sur un réservoir de $CO_2$ de façon à contrôler la libération du $CO_2$ à l'intérieur de ladite enceinte.

Ce dispositif permet en faisant varier la teneur en $CO_2$ dans le milieu de culture, de faire varier également le pH de ce milieu. L'augmentation de la teneur en $CO_2$ et la diminution du pH qui en découle provoque un "stress" de la culture de micro-organismes. Les cellules de micro-organismes relâchent alors dans l'effluent et le milieu de culture liquide, les polluants qu'elles avaient piégés. On obtient ainsi le résidu de polluants concentrés.

Enfin, l'invention concerne également un procédé d'épuration d'un effluent liquide chargé en polluants, notamment en métaux et/ou en radionucléïdes.

Selon les caractéristiques de l'invention, ce procédé comprend les étapes consistant à :

3

- faire passer cet effluent au travers d'un support dans lequel sont immobilisées des cellules vivantes de micro-organismes photosynthétiques aptes à piéger lesdits polluants,
- récupérer après le passage sur ce support, l'effluent traité liquide obtenu, et
- agir ponctuellement sur les cellules vivantes de micro-organismes pour les forcer à libérer dans le milieu circulant les polluants piégés, de façon à obtenir un résidu de polluants concentrés.

La technique consistant à immobiliser les cellules vivantes à l'intérieur d'un support est particulièrement avantageuse, car les polluants sont piégés dans les parois ou même à l'intérieur des cellules vivantes et ne sont élués dans le milieu liquide circulant qu'à certaines périodes, sans que les cellules vivantes soient présentes dans ce milieu liquide. En conséquence, il n'est plus nécessaire d'effectuer une étape de séparation du solide et du liquide et l'on simplifie ainsi grandement le procédé d'épuration des effluents.

L'invention sera mieux comprise à la lecture de la description suivante d'un mode de réalisation de l'invention, donné à titre d'exemple illustratif et non limitatif, cette description étant faite en faisant référence aux dessins joints, dans lesquels :
- la figure 1 est une vue en coupe d'un dispositif d'épuration d'un effluent liquide selon l'invention, et
- la figure 2 est un schéma illustrant un système global d'épuration dans lequel plusieurs dispositifs d'épuration selon la figure 1 sont montés en parallèle.

Le dispositif d'épuration selon l'invention permet de traiter un effluent chargé notamment en métaux, (y compris les métaux lourds) et/ou en radionucléides.

Le dispositif selon l'invention comprend une enceinte de confinement 1 réalisée dans un matériau transparent aux rayonnements lumineux. Ce matériau peut être par exemple le verre, le polycarbonate, le polyméthacrylate ou le PVC (polychlorure de vinyle). La source lumineuse placée autour de l'enceinte de confinement 1 peut être soit naturelle, soit artificielle. Cette enceinte 1 présente une forme sensiblement cylindrique. Elle est conique dans sa partie basse 3 et son extrémité supérieure est avantageusement fermée par un couvercle 5 également réalisé dans un matériau transparent à la lumière. Bien que ce couvercle 5 ne soit pas obligatoire, il permet de conserver une température constante à l'intérieur de l'enceinte et de limiter les risques de pollution externe.

Cette enceinte 1 contient un support 7 à l'intérieur duquel sont immobilisées des cellules vivantes de micro-organismes photosynthétiques. Ces cellules vivantes sont choisies parmi les bactéries, les micro-algues ou les cellules isolées de végétaux supérieurs. Dans la suite de la description, pour simplifier on parlera uniquement de cellules de micro-algues.

Le support 7 est avantageusement réalisé dans un matériau également transparent aux rayonnements lumineux, par exemple la mousse de polyuréthane. De façon avantageuse, ce support 7 se présente sous forme de fragments de mousse de polyuréthane, de préférence de petits dés dont les côtés mesurent par exemple 1,5 cm environ. Ce support est traversé simultanément par l'effluent liquide à traiter et par un milieu de culture liquide, comme cela sera décrit ultérieurement.

La fragmentation du support permet d'obtenir une granulométrie offrant simultanément un bon compromis entre la nécessité de maintenir une bonne circulation du milieu de culture, comme cela sera décrit ultérieurement et la nécessité d'obtenir une bonne diffusion de l'effluent à traiter jusqu'au niveau des cellules des micro-algues immobilisées dans le support.

L'immobilisation des cellules vivantes de micro-algues à l'intérieur de la mousse de polyuréthane peut s'effectuer soit en piégeant les cellules au cours de la polymérisation d'un prépolymère d'uréthane, soit par colonisation de la mousse de polyuréthane, par les cellules de micro-algues, depuis l'extérieur. Le brevet FR 2 547 823 décrit justement un exemple de procédé de préparation d'une paroi transparente réalisée en mousse de polyuréthane et dans les pores de laquelle sont répartis des micro-organismes. Ce procédé consiste à introduire dans un moule, un mélange comprenant des précurseurs de mousse de polyuréthane et une suspension de micro-organismes et à faire polymériser l'ensemble.

Le support d'immobilisation 7 contenant les cellules vivantes de micro-algues est maintenu dans un espace déterminé à l'intérieur de l'enceinte de confinement 1 grâce à une grille inférieure 9 et à une grille supérieure 11. En outre, on prévoit à l'intérieur de l'enceinte de confinement 1 et plus précisément, du support 7, un puits 13 formant trop-plein. De façon avantageuse, ce puits 13 est placé au centre de l'enceinte de confinement 1.

La grille supérieure 11 permet d'éviter que les fragments de support 7 ne tombent dans le puits central 13 tandis que la grille inférieure 9 permet d'assurer une séparation franche entre le support d'immobilisation 7 et le milieu liquide circulant à l'intérieur de l'enceinte. En conséquence, le support d'immobilisation 7 ne s'étend que sur une partie de la hauteur totale de l'enceinte de confinement 1, de façon à ménager un espace libre inférieur 10 et un espace libre supérieure 12 à l'intérieur de ladite enceinte.

Le puits central 13 s'étend entre ces deux espaces libres. Il est avantageusement vertical.

De préférence, les parois 15 constituant ce puits sont doubles de façon à permettre la circulation, entre elles, d'un liquide de régulation thermique. Celui-ci est introduit à la partie basse du puits 13 par une canalisation d'entrée 17 et sort par une canalisation de sortie 19, prévue dans la partie haute du puits 13. Ce liquide de régulation thermique permet d'assurer que le milieu de culture liquide circulant à l'intérieur de l'enceinte de confinement 1 est maintenu à une température constante correspondant à la température de culture optimale des micro-algues.

Ce milieu de culture indispensable au développement des cellules vivantes de micro-algues est introduit par des moyens 21 constitués par une canalisation prévue au travers du couvercle 5 et débouchant avantageusement au-dessus du puits 13. Le dispositif d'épuration comprend en outre des moyens 23 de mise en circulation de ce milieu de culture à l'intérieur de l'enceinte. Ces moyens peuvent être une pompe. Toutefois, une autre variante de réalisation de ces moyens sera décrite ultérieurement.

L'extrémité conique 25 de la partie inférieure 3 de l'enceinte de confinement est munie d'une canalisation en T 27. Sur la partie centrale de cette canalisation en T 27, est prévue une vanne d'entrée/sortie 29, tandis que sur l'une des branches est prévue une vanne d'entrée 31 de l'effluent à traiter et sur l'autre branche une vanne de sortie 33. Le dispositif selon l'invention comprend également des moyens 35 de mise en circulation de l'effluent à traiter. Ces moyens 35 peuvent être une pompe, par exemple.

En pratique, les moyens de mise en circulation 23 du milieu de culture et 35 de l'effluent à traiter sont confondus puisque le milieu de culture et l'effluent se mélangent à l'intérieur de l'enceinte. De façon avantageuse, ces moyens 23 et 35 sont constitués par au moins un injecteur de gaz placé dans la partie conique 3 de l'enceinte et qui permet de faire circuler la masse liquide totale de bas en haut de l'enceinte de confinement et du support. Le mélange gazeux introduit est constitué d'air et avantageusement de $CO_2$. De préférence, le mélange comprend entre 1 et 10% de $CO_2$ et mieux 2% de $CO_2$. Le $CO_2$ introduit favorise la photosynthèse. L'air est stocké dans un réservoir 37 commandé par une vanne 39 et le $CO_2$ dans un réservoir 41 commandé par une vanne motorisée (ou électrovanne) 43.

Les bulles de gaz remontent à travers le support d'immobilisation 7 et entraînent le milieu de culture et l'effluent à traiter qui sont donc en contact maximum avec les cellules vivantes des micro-algues. Ainsi entraîné, le milieu liquide arrive au sommet de l'enceinte 1, dans l'espace libre supérieur 12. Ce principe de circulation est connu sous le terme anglais de "air-lift", ou français "d'ascenseur à gaz". Le couvercle 5 est en outre muni d'orifices 45 permettant le dégazage de l'enceinte de confinement.

Comme cela a déjà été expliqué précédemment rapidement, les éléments polluants, c'est-à-dire les métaux et les radionucléïdes, piégés par les cellules vivantes de micro-algues immobilisées dans le support 7, peuvent être désorbés sous l'action d'un stress et en particulier sous l'action d'un choc de pH, ou encore sous l'action d'un choc osmotique. Ce dernier peut être provoqué en modifiant la salinité du milieu liquide dans lequel baigne le support d'immobilisation 7. Une sonde de mesure du pH 47 est placée dans l'une des parois latérales de l'enceinte de confinement 1. Cette sonde de pH 47 est reliée à un analyseur de pH 49 lui-même relié à un régulateur de pH 51. En fonction d'une valeur de référence, le pH peut être régulé par l'action de l'électro-vanne ou vanne motorisée 43 connectée au régulateur de pH 51 d'une part, et au récipient de stockage 41 de gaz carbonique d'autre part. Comme cela a été précédemment décrit, ce réservoir de stockage 41 débouche par une canalisation dans l'éjecteur 23, 35.

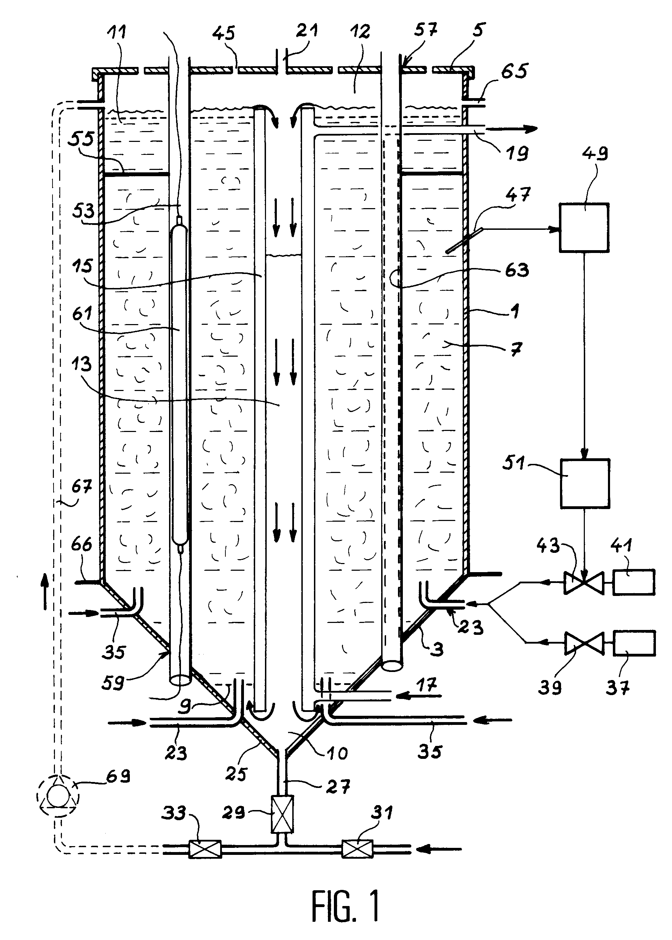

A des fins de simplification de la figure 1, un seul circuit de régulation du pH a été représenté, toutefois, tous les injecteurs 23, 35 sont reliés à un circuit de régulation ou au moins à un circuit de régulation commun. Ainsi, il est possible soit d'optimiser la photosynthèse des micro-algues en ajustant le pH et le $CO_2$ à une valeur de culture optimum, soit d'abaisser fortement ce pH de façon à exercer un stress sur les cellules vivantes et à les forcer ainsi à désorber dans le milieu liquide les métaux et autres polluants accumulés.

En outre de façon avantageuse, l'enceinte de confinement 1 peut être munie d'au moins un tube transparent 53 la traversant sensiblement verticalement. De préférence, elle comprend deux tubes. Chaque tube 53 est fixé sur la face interne des parois de l'enceinte de confinement par une entretoise 55. En outre, le couvercle 5 est muni d'une ouverture 57 destinée à laisser le passage à ce tube 53 et pour les mêmes raisons la partie inférieure 3 de l'enceinte est munie d'une ouverture 59. Ces tubes 53 sont ouverts à leurs deux extrémités. Ils sont destinés à favoriser la pénétration de la lumière naturelle à l'intérieur de l'enceinte de confinement 1 ou à recevoir une source de lumière artificielle telle qu'une fibre optique ou un tube fluorescent 61, comme cela est réprésenté dans la partie gauche de la figure 1. De plus, l'ouverture de chaque tube 53 à ses deux extrémités permet d'améliorer le contrôle thermique de l'enceinte 1 et d'évacuer par convexion une grande quantité des calories apportées, par exemple, par une source de lumière artifielle.

De façon avantageuse, la paroi interne de ces tubes transparents 53 peut être recouverte d'un film sélectif 63 permettant de privilégier la prédominance de certaines longueurs d'onde. Ce film 63 a été partiellement représenté sur le tube 53 de droite de la figure 1. En conséquence, chacun de ces tubes transparents 53 permet de modifier la durée de la photopériode, la quantité de lumière reçue et le qualité spectrale de cette lumière.

En outre, l'enceinte de confinement 1 est munie d'au moins une canalisation de surverse 65, prévue dans l'espace libre supérieur 12 de l'enceinte, de façon à affleurer au niveau du milieu liquide et à constituer un trop-plein.

Enfin, on notera que l'enceinte 1 est munie à sa base d'un support annulaire 66 permettant de la maintenir verticalement.

Le fonctionnement de ce dispositif d'épuration va maintenant être expliqué plus en détail.

Les cellules vivantes des micro-algues sont piégées à l'intérieur de la mousse de polyuréthane comme cela a été décrit précédemment et les fragments de mousse sont introduits à l'intérieur de l'enceinte de confinement. Le milieu de culture liquide enrichi en azote est également introduit dans l'enceinte par la canalisation 21, afin que la culture puisse croître et se multiplier. Du $CO_2$ est également ajouté.

En présence de lumière et de $CO_2$, les micro-algues effectuent une réaction de photosynthèse au cours de laquelle elles transforment le gaz carbonique en oxygène et forment de la biomasse. Cette réaction biochimique correspond à une photopolymérisation du gaz carbonique ; elle peut être schématisée par l'équation de MYERS suivante :

$$6,14\ CO_2 + 3,65\ H_2O + NH_3 \rightarrow C_{6,14}H_{10,3}O_{2,2} + 6,85\ O_2$$

L'oxygène produit peut éventuellement être récupéré pour être utilisé dans d'autres applications. L'utilisation de la mousse de polyuréthane transparente à la lumière permet donc d'effectuer cette culture de micro-algues dans des conditions photo-autotrophiques vis-à-vis du $CO_2$.

Le choix des espèces de micro-algues à immobiliser dépend grandement de la nature de l'effluent à épurer. Toutefois, dans un mode de réalisation préféré de l'invention, ce sont des micro-algues de l'espèce Porphyridium cruentum qui ont été utilisées. Les micro-algues de cette espèce sont capables de produire des polysaccharides exocellulaires.

Le tableau 1 ci-après donne quelques exemples des micro-algues qu'il est possible de cultiver à l'intérieur du dispositif d'épuration selon l'invention, en fonction des métaux ou radionucléides contenus dans les effluents que l'on souhaite traiter.

Tableau 1

| Espèces de micro-algues | Eléments pouvant être piégés par cette micro-algue |
|---|---|
| Chlorella vulgaris | cuivre, or |
| Chlorella sp | mercure, uranium |
| Chlorella régularis | manganèse, molybdène, uranium |
| Scenedesmus | cadminium, molybdène, uranium |
| Euglena sp | fer, aluminium, baryum, zinc, manganèse, nickel, cuivre, plomb, uranium, thorium |

6

Bien entendu, cette liste n'est pas exhaustive et il est possible de cultiver d'autres espèces de micro-algues à l'intérieur du dispositif selon l'invention.

Dans le cas d'espèces produisant des polysaccharides telle que Porphyridium cruentum utilisée ici et lorsque les cellules de micro-algues sont arrivées à un stade de développement suffisant pour coloniser la majeure partie du support d'immobilisation 7, l'apport d'azote est supprimé ou fortement réduit. Les micro-algues réagissent alors et se protègent en synthétisant un polysaccharide exocellulaire qui reste en partie accroché à la surface extérieure de la cellule et dont l'autre partie est hydrosolubilisé dans le milieu liquide entourant les supports 7. Ce sont sur ces polysaccharides que vont se fixer les ions métalliques ou les radionucléides.

D'autres espèces de micro-algues telles que Chlorella ou Scenedesmus peuvent être cultivées dans le biofiltre en présence d'azote. Dans ce cas, les micro-algues produisent peu ou très peu de polysaccharides et les éléments polluants sont accumulés à l'intérieur de la cellule algale.

Le mode de concentration des métaux ou des radionucléides dépend de l'espèce d'algue cultivée et de cette espèce dépendra donc le mode de récupération des éléments concentrés et le mode de fonctionnement du biofiltre.

Dans le cas d'une souche d'algue comme Porphyridium cruentum produisant un polysaccharide exocellulaire, le stockage est externe à la cellule. Dans ce cas, le biofiltre est rempli avec l'effluent à épurer par l'intermédiaire des vannes 29 et 31, et cet effluent circule dans le biofiltre. Comme décrit précédemment, les ions considérés comme polluant (métaux lourds, radionucléides, ...) se fixent sur le polysaccharide formant ainsi un complexe électriquement neutre. On considère comme épuré un effluent qui ne contient plus ou pratiquement plus de polluant sous forme ionique, c'est-à-dire un effluent dont la totalité des ions a été fixée sur le polysaccharide. L'état de dépollution peut être vérifié en précipitant le polysaccharide et donc avec lui les ions polluants fixés, par exemple, en ajoutant de l'alcool à un échantillon de l'effluent. Le dosage de l'ion polluant dans le surnageant après précipitation permet alors de déterminer, par rapport à la quantité initialement présente dans l'effluent, quelle est la proportion d'ions fixés sur le polysaccharide et ainsi le degré d'épuration de cet effluent. Dès que la proportion d'ions résiduels dans l'effluent est nulle ou très faible, cet effluent est élué par les vannes 29 et 33. L'effluent ainsi récupéré est traité avec un alcool par exemple de façon à faire précipiter les polysaccharides exocellulaires hydrosolubilisés et ainsi à récupérer les éléments polluants, tels que les métaux et les radionucléides.

Dans le cas d'une souche comme Chlorella où le stockage de l'élément à éliminer est intracellulaire, le mode de fonctionnement est légèrement différent. Le biofiltre est rempli par les vannes 29 et 31, avec l'effluent à épurer. Au bout d'un temps suffisant pour épurer complètement l'effluent, l'effluent traité liquide obtenu est élué par l'intermédiaire des vannes 29 et 33. Ces opérations de remplissage du biofiltre et d'élution sont répétées plusieurs fois. L'effluent est élué et le biofiltre est de nouveau rempli dès que la concentration en métaux ou en radionucléides de l'effluent à traiter est nulle et ceci jusqu'à ce que les micro-algues soient saturées (concentration intracellulaire maximale). Cette saturation intervient lorsque la concentration de l'effluent en éléments chimiques à éliminer ne diminue plus.

A ce moment là, sous l'action d'un choc de pH ou d'une modification de la pression osmotique ou d'autres paramètres physico-chimiques, les éléments polluants concentrés dans la cellule algale sont excrétés dans le milieu liquide entourant le support d'immobilisation 7. Le biofiltre est alors élué une dernière fois par les vannes 29 et 33 et le liquide obtenu correspond à un concentré des éléments chimiques à éliminer. Le facteur de concentration réalisé sera fonction du nombre de remplissage et d'élution qui aura été effectué.

Les deux modes de fonctionnement ainsi décrits sont des modes de fonctionnement dit en "batch" ou "semi continu", c'est-à-dire qu'on charge l'effluent et qu'on le soutire uniquement lorsque la totalité des éléments polluants sont éliminés de cet effluent.

Il existe un mode de fonctionnement dit en continu qui est applicable quelque soit l'espèce d'algue choisie. Dans ce cas, le biofiltre est rempli une première fois avec l'effluent par les vannes 29 et 31, mais au lieu d'être périodiquement vidé puis rechargé, cet effluent est ensuite apporté en continu via l'orifice 21 par une micropompe. Du débit de cette micropompe dépend le taux de dilution et le temps de séjour moyen de cet effluent dans le biofiltre et donc du degré de purification de l'effluent. Une quantité de liquide équivalente à celle introduite est soutirée par la canalisation de surverse 65 ou à l'aide d'une deuxième micropompe. On notera que la position de la canalisation 21 (au milieu du couvercle 5) est judicieuse, car elle permet d'éviter que l'effluent apporté soit éventuellement immédiatement soutiré par la canalisation de surverse 65.

Dans le cas d'un stockage intracellulaire, l'effluent ainsi récolté est considéré comme épuré. Lorsque la concentration en élément polluant augmente dans le milieu liquide contenu dans le biofiltre, les algues sont alors "saturées" en cet élément. Ce dernier est alors récupéré comme précédemment décrit par un choc osmotique ou de pH.

Dans le cas d'un stockage extracellulaire sur un polysaccharide, l'effluent récolté est traité à l'alcool par exemple comme décrit précédemment. Dans ce cas particulier, il est possible d'apporter, en même temps que l'effluent, certains éléments nutritifs favorisant la synthèse des polysaccharides. La production de ces polysaccharides est alors constante dans le temps ; ceci permettant de maintenir un mode de fonctionnement en continu sur de longues périodes.

Par ailleurs, dans le cas d'une absence d'aération ou d'un milieu de culture trop visqueux, il est possible de recycler l'effluent à traiter en le prélevant au niveau de la vanne de sortie 33 et en l'introduisant de nouveau à l'intérieur de l'enceinte de confinement, dans l'espace libre supérieur 12, par une canalisation de recirculation 67. Cette canalisation 67 est munie d'une pompe 69. Cette variante facultative a été illustrée en traits mixtes en figure 1.

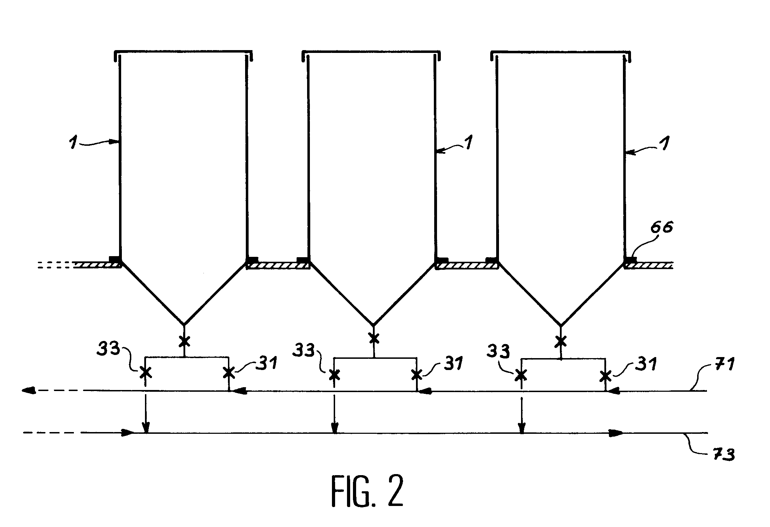

Enfin, comme illustré en figure 2, plusieurs dispositifs d'épuration selon l'invention peuvent être montés en parallèle. Toutes les vannes d'entrée 31 sont montées sur une canalisation commune d'entrée 71 et toutes les vannes de sortie 33 sur une canalisation commune de sortie 73.

## Revendications

1. Dispositif d'épuration d'un effluent liquide chargé en polluants, notamment en métaux et/ou en radionucléïdes, comprenant une enceinte de confinement (1), destinée à recevoir des cellules de micro-organismes photosynthétiques, aptes à piéger lesdits polluants, des moyens (31) d'introduction de l'effluent à traiter, des moyens (35) de mise en circulation de cet effluent à travers ladite enceinte (1), des moyens de prélèvement (33, 65) de l'effluent traité liquide et des moyens de prélèvement (33) des polluants, et des moyens (23) d'introduction de $CO_2$ à l'intérieur de l'enceinte, caractérisé en ce que les cellules de micro-organismes sont des cellules vivantes, immobilisées sur un support (7), ce support (7) occupant au moins partiellement l'intérieur de l'enceinte (1) et étant traversé par l'effluent à traiter et en ce que le dispositif comprend des moyens d'introduction (21) et des moyens de mise en circulation (23) d'un milieu de culture nutritif liquide à travers ledit support (7), les parois de l'enceinte (1) ainsi que le support (7) des cellules étant réalisés dans un matériau transparent aux rayonnements lumineux.

2. Dispositif d'épuration selon la revendication 1, caractérisé en ce que les moyens de mise en circulation (23, 35) assurent le déplacement de l'effluent et du milieu de culture de bas en haut du support d'immobilisation (7) des cellules, en ce que ledit support (7) ne s'étend que sur une partie de la hauteur totale de l'enceinte de confinement (1), de façon à ménager un espace libre inférieur (10) et un espace libre supérieur (12) à l'intérieur de ladite enceinte et en ce qu'un puits (13) formant trop-plein est prévu dans le support (7) pour relier entre eux lesdits deux espaces libres (10, 12), le milieu de culture et l'effluent se déversant dans ce puits (13) après être parvenus dans l'espace libre supérieur (12).

3. Dispositif d'épuration selon la revendication 1 ou 2, caractérisé en ce que les moyens de mise en circulation (35) de l'effluent et (23) du milieu de culture comprennent un injecteur relié à un réservoir d'air (37) et permettant d'injecter des bulles d'air dans la partie inférieure de l'enceinte (1).

4. Dispositif d'épuration selon les revendications 1 et 3, caractérisé en ce que l'injecteur (23, 35) est également relié à un réservoir de $CO_2$ (41) et permet d'injecter de l'air contenant de 1 à 10% de $CO_2$.

5. Dispositif d'épuration selon la revendication 1 ou 4, caractérisé en ce qu'il comprend, montés en série, un capteur de mesure du pH (47) régnant à l'intérieur de l'enceinte de confinement (1), un analyseur de pH (49), un régulateur de pH (51) et une vanne (43) agissant sur un réservoir de $CO_2$ (41), de façon à contrôler la libération du $CO_2$ à l'intérieur de ladite enceinte.

6. Dispositif d'épuration selon la revendication 2, caractérisé en ce que les parois (15) du puits (13) sont doubles de façon à permettre à l'intérieur, la circulation d'un fluide de régulation thermique.

7. Dispositif d'épuration selon l'une quelconque des revendications précédentes, caractérisé en ce qu'au moins un tube (53) est placé à l'intérieur de l'enceinte de confinement et traverse celle-ci de part en part, ce tube permettant le passage des rayons lumineux provenant de l'extérieur.

8. Dispositif d'épuration selon la revendication 7, caractérisé en ce qu'une lampe (61) est placée à l'intérieur dudit tube (53).

9. Dispositif d'épuration selon la revendication 7 ou 8, caractérisé en ce qu'un film (63) permettant de sélectionner le passage de longueurs d'onde prédéterminées recouvre les parois internes du tube (53).

**10.** Dispositif d'épuration selon les revendications 2 et l'une des revendications 7 à 9, caractérisé en ce que le puits (13) formant trop-plein est placé au centre de l'enceinte de confinement (1) et en ce qu'au moins deux tubes (53) sont répartis uniformément autour.

**11.** Dispositif d'épuration selon la revendication 1, caractérisé en ce que les moyens de prélèvement (65) de l'effluent traité liquide comprennent une canalisation de surverse placée au sommet de l'enceinte de confinement (1).

**12.** Dispositif d'épuration selon la revendication 2, caractérisé en ce que les moyens de prélèvement (33) sont reliés par une canalisation (67) munie d'une pompe (69) à l'espace libre supérieur (13) de l'enceinte de confinement.

**13.** Dispositif d'épuration selon la revendication 1, caractérisé en ce que le support d'immobilisation (7) comprend des fragments de mousse de polyuréthane.

**14.** Dispositif d'épuration selon la revendication 1, caractérisé en ce que le micro-organisme photosynthétique est une micro-algue, une bactérie ou une cellule isolée de végétaux supérieurs.

**15.** Dispositif d'épuration selon la revendication 14, caractérisé en ce que la micro-algue est de l'espèce Porphyridium cruentum et produit un polysaccharide exocellulaire.

**16.** Procédé d'épuration d'un effluent liquide chargé en polluants, notamment en métaux et/ou en radionucléides, caractérisé en ce qu'il comprend les étapes consistant à :
- faire passer cet effluent au travers d'un support (7) dans lequel sont immobilisées des cellules vivantes de micro-organismes photosynthétiques aptes à piéger lesdits polluants,
- récupérer après le passage sur ce support (7), l'effluent traité liquide obtenu, et
- agir ponctuellement sur les cellules vivantes de micro-organismes pour les forcer à libérer dans le milieu circulant les polluants piégés, de façon à obtenir un résidu de polluants concentrés.

**17.** Procédé d'épuration selon la revendication 16, caractérisé en ce que l'action ponctuelle sur les cellules vivantes consiste à abaisser le pH du milieu liquide dans lequel baigne le support d'immobilisation (7).

**18.** Procédé d'épuration selon la revendication 16, caractérisé en ce que l'action ponctuelle sur les cellules vivantes consiste à provoquer un choc osmotique en modifiant la salinité du milieu dans lequel baigne le support d'immobilisation (7).

**19.** Procédé d'épuration selon la revendication 16, caractérisé en ce que l'action ponctuelle sur les cellules vivantes consiste à réduire le taux d'azote du milieu liquide dans lequel baigne le support d'immobilisation (7).

**20.** Procédé d'épuration selon l'une quelconque des revendications précédentes, caractérisé en ce que les cellules vivantes de micro-organismes sont des micro-algues de l'espèce Porphyridium cruentum.

FIG. 1

FIG. 2

EP 0 599 711 A1

Office européen
des brevets

**RAPPORT DE RECHERCHE EUROPEENNE**

Numero de la demande

EP 93 40 2818

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| Y | FR-A-1 513 913 (UNION TANK CAR COMPANY) * page 4, colonne de droite, ligne 13 - ligne 37 * * page 5, colonne de gauche, ligne 14 - ligne 24 * | 1,7,8,14 | C02F3/32 C12M1/00 |
| Y A | WO-A-91 07080 (MARTEK CORPORATION) * page 5, ligne 6 - ligne 20 * * page 8, ligne 16 - ligne 23 * * page 11, ligne 26 - page 12, ligne 8 * | 1,7,8,14 5 | |
| Y | FR-A-2 162 465 (BIO-KINETICS INC.) * page 2, ligne 23 - ligne 37 * * page 3, ligne 31 - ligne 40; revendication 6 * | 1,3,4,7, 8,14-16, 20 | |
| Y | EP-A-0 112 556 (MORI,KEI) * page 2, ligne 6 - page 3, ligne 19 * | 1,3,4,7, 8,14-16, 20 | **DOMAINES TECHNIQUES RECHERCHES (Int.Cl.5)** C02F C12M |
| A | PATENT ABSTRACTS OF JAPAN vol. 9, no. 66 (C-271)26 Mars 1985 & JP-A-59 199 099 (ICHIGORDU SEKINE) 12 Novembre 1984 * abrégé * | 1,14,16 | |
| A,D | EP-A-0 130 116 (COMMISSARIAT A L'ENERGIE ATOMIQUE) * abrégé * | 13 | |

Le présent rapport a été établi pour toutes les revendications

| Lieu de la recherche | Date d'achèvement de la recherche | Examinateur |
|---|---|---|
| LA HAYE | 1 Février 1994 | Gonzalez Arias, M |

EPO FORM 1503 03.82 (P04C02)